# EUROPEAN PATENT APPLICATION

(11) **EP 4 726 053 A1**
(43) Date of publication of application: **15.04.2026**
(21) Application number: 25761199.6
(22) Date of filing: 25.02.2025
(51) Int. Cl.: C12Q 1/02, C12N 5/071, C12N 13/00

(54) **METHOD FOR MEASURING ELECTRIC SIGNAL OF CELL TISSUE, AND SENSITIZER**

(30) Priority: 29.02.2024 JP 2024029363
(71) Applicant: The Foundation for the Promotion of Industrial Science, Tokyo 153-8505 (JP)
(72) Inventor: IKEUCHI Yoshiho, Tokyo 153-8505 (JP); DUENKI Tomoya, Tokyo 153-8505 (JP)
(74) Representative: Müller-Boré & Partner Patentanwälte PartG mbB
(86) International application number: PCT/JP2025/006202
(87) International publication number: WO 2025/182853

(57) **Abstract**

The purpose of the present invention is to easily and efficiently obtain an electric signal generated in cell tissue by increasing the electric signal obtained from the cell tissue in a state in which there is no damage to cells and observation with a microscope or the like is also possible. The present invention pertains to: a method for measuring an electric signal of cell tissue by adding a perfluorocarbon compound having at least 5 carbon atoms; and an electric signal enhancer for cell tissue, comprising a perfluorocarbon compound having at least 5 carbon atoms. Moreover, provided are: a method for measuring an electric signal of cell tissue, wherein the perfluorocarbon compound is preferably perfluorodecalin, the measurement of the electric signal of the cell tissue is preferably performed by means of an electrode array, and the addition of the perfluorocarbon compound is preferably an addition caused by substituting a culture solution; and an electric signal enhancer for cell tissue.

## Description

### Technical Field

The present invention relates to a method for measuring electric signals of cell tissue and an enhancer.

### Background Art

A method for elucidating the functions of cell tissue includes measuring electric signals generated in cell tissue.

There are two well-known methods for measuring electric signals generated in cell tissue: the patch clamp method, which includes measuring electric signals of cell tissue outside the cell tissue, and a method including inserting electrodes inside the cell tissue to measure electric signals.

Recently, the method for electrical measurement of cell tissue using microelectrode arrays (hereinafter referred to as "MEA") has become widely known (Patent Literature 1, Patent Literature 2). This method is a so-called non-invasive method, in which measurements is made outside of cell tissue, and is also capable of observing many locations in the tissue simultaneously over a long period of time. It is possible to measure electric signals generated in cell tissue using electrodes densely arranged in an array. Furthermore, this method has made a significant contribution to elucidating the functions of cell tissues through the development of high-density microelectrode arrays (hereinafter referred to as "HD-MEA"), which are based on solid-state imaging device technology using CMOS (Complementary Metal Oxide Semiconductor) (Non Patent Literature 1). This electrode array has extremely high spatial and temporal resolution, making it possible to observe the activities of cell tissue in detail.

Any cell tissue can be targeted as a cell tissue from which electric signals can be measured, and representative examples include neurons and cardiomyocytes. Recently, in view of usefulness of measuring electric signals, the importance of in vitro electric signal measurement has been increasing particularly for cell tissue of primary cells, neurons and the like, derived from human induced pluripotent stem cells (hereinafter referred to as "hiPSCs"). These cell tissues range from simple 2-dimensional cell tissues consisting of a single cell tissue to 3-dimensional cell tissues with complex cellular structures, and electric signals can be measured regardless of the shape of the cell tissue.

When measurement is made on 3-dimensional cell tissues, planar MEAs designed for single cell tissues have a limited area of contact with the 3-dimensional cell tissues. Therefore, various electrode designs, such as shell-like electrodes, stretchable mesh nanostructured electrodes, and kirigami-structured electrodes which transition from 2-dimensional to 3-dimensional basket-like structures, have been reported to enable and further improve 3-dimensional recording for neural organoids and the like, which generally have a round morphology. Thus, the continued improvement of fabrication techniques for neural tissues such as organoids and advances in electrode technology for cell research are increasing the usefulness of electrophysiological testing. This advancement is expected to elucidate fundamental principles of brain function, provide insight into neurological diseases, and facilitate the discovery of novel treatments.

In particular, 3-dimensional tissues such as neural organoids have attracted great interest because they can be derived from human induced pluripotent stem cells and can recapitulate important aspects of the brain.

However, the above-mentioned electrodes are complicated and therefore difficult to manufacture, and are also difficult to handle. Furthermore, observation using a microscope or the like is impossible. Therefore, there has been a demand for a method for measuring electric signals which is simple, causes minimal damage to cell tissue, and furthermore, enables observation using a microscope or the like, but such a convenient method has not existed until now.

On the other hand, from a completely different perspective, a substance called perfluorodecalin (hereinafter referred to as "PFD"), which is as follows, is known in the field of biotechnology:
PFD consists of 10 carbon atoms and 18 fluorine atoms, and is characterized by its high hydrophobicity and chemical stability. As fully fluorinated, PFD is immiscible with water and is known to function as effective oxygen carrier. Furthermore, PFD is colorless and chemically and biologically inert. These unique properties make PFD a compelling candidate substance for various biotechnological applications across a wide range of biotechnology fields. For example, PFD-based components have been studied for locally increasing oxygen supply to increase wound healing such as in second-degree burns in porcine skins as well as in acute ocular chemical burns. It has also been shown to enhance short-term tissue preservation effect in organ isolation and transplantation. Furthermore, it is widely used in biomedical imaging as a contrast agent in magnetic resonance imaging (MRI), ultrasound imaging and the like. Furthermore, PFD-based nanoparticles are expected to be applied to drug delivery systems, and are being used to encapsulate hydrophobic drugs, with their solubility and release control attracting attention.

Furthermore, perfluorohexane (hereinafter referred to as "PFH"), perfluoroheptane (hereinafter referred to as "PFHept"), perfluorooctane (hereinafter referred to as "PFO") and the like are well known as similar substances to PFD.

Liquid at normal temperature or at a culture temperature of around 37°C is preferred.

### Citation List

### Patent Literature

Patent Literature 1: International Publication No. WO2015/012955
Patent Literature 2: International Publication No. WO2020/189172

### Non Patent Literature

Non Patent Literature 1: Muller, J. et al. High-resolution CMOS MEA platform to study neurons at subcellular, cellular, and network levels. Lab Chip 15, 2767-2780 (2015)

### Summary of Invention

### Technical Problem

The object of the present invention is to easily and efficiently obtain electric signals generated in cell tissue.

### Solution to Problem

The electric signals emitted by cell tissue are weak, and the measurable electric signals are also small. Furthermore, existing electrodes for obtaining large amounts of electric signals are difficult to handle and makes observation using a microscope or the like impossible.

The present inventors have surprisingly found that, when measuring electric signals of cell tissue, addition of a perfluorocarbon compound having 5 or more carbon atoms, which is a chemical substance, makes it possible to increase the electric signal obtained from the cell tissue, with minimal damage to the cells, in a state in which observation with a microscope or the like is also possible, and to easily and efficiently obtain electric signals generated in cell tissue, thereby completing the present invention.

Specifically, the present invention is characterized by the following points:
1. A method for measuring electric signals of cell tissue, comprising adding a perfluorocarbon compound having 5 or more carbon atoms and measuring electric signals of the cell tissue.
2. The method for measuring electric signals of cell tissue according to 1 above, wherein the perfluorocarbon compound is perfluorodecalin.
3. The method for measuring electric signals of cell tissue according to 1 or 2 above, wherein the measurement of electric signals of cell tissue is performed using an electrode array.
4. The method for measuring electric signals of cell tissues according to 1 or 2 above, wherein the adding the perfluorocarbon compound is adding by replacing a culture solution.
5. An electric signal enhancer for cell tissues, comprising a perfluorocarbon compound having 5 or more carbon atoms.
6. The electric signal enhancer for cell tissue according to 5 above, wherein the perfluorocarbon compound is perfluorodecalin.

### Advantageous Effects of Invention

As shown in the Examples of the present application, addition of a perfluorocarbon compound having 5 or more carbon atoms can increase the sensitivity in measuring electric signals generated in cell tissue to easily and efficiently measure electric signals generated in cell tissue, without damaging the cell tissue, in a state which allows observation using a microscope or the like.

### Brief Description of Drawings

[Figure 1A] Comparative images of primary mouse hippocampal cells (in vitro, day 7) with and without PFD addition. Scale bar is 250 µm. "Control" is a comparative example before PFD addition.
[Figure 1B] Comparison of cell surface area (µm²). The area increases with PFD addition.
[Figure 1C] Images of 3-dimensional reconstructions of Z-stacks of primary hippocampal cells stained with calcein AM, acquired using a confocal imaging system, by comparison with and without PFD addition. Scale bar is 20 µm.
[Figure 1D] The heights (µm) of the cell bodies is flattened under PFD condition.
[Figure 2A] From top to bottom: comparison of active electrode maps of the chip with and without PFD addition (the "1" side is active), comparison of mean spike amplitude maps of the chip with and without PFD addition (units: µV), comparison of mean spike rate maps of the chip with and without PFD addition (units: Hz).
[Figure 2B] The number of detected active electrodes is significantly greater with PFD addition. "Washed" indicates the results after PFD is removed.
[Figure 2C] The histogram of spike amplitude (µV) at the active electrodes with PFD addition shows a distribution shifted to higher amplitudes under PFD condition.
[Figure 2D] The mean spike amplitude (µV) of the active electrodes is significantly higher under PFD condition.
[Figure 2E] Histogram comparison of the mean spike rate (Hz) of active electrodes with and without PFD addition. The rate is significantly higher under PFD condition.
[Figure 2F] The mean spike rate (Hz) of active electrodes is significantly higher with PFD addition under PFD condition.
[Figure 2G] Changes in hippocampal cell activity with and without PFD addition.
[Figure 3A] Comparative images of cortical organoids with and without PFD addition.
[Figure 3B] PFD addition increases the organoid diameter (mm), which reverts after washing out PFD.
[Figure 3C] Confocal comparison images of organoids at the focal plane of the culture dish bottom with and without PFD addition. Scale bar is 100 µm.
[Figure 3D] Comparison of contact area (mm²) with the bottom surface of the organoid. PFD addition increases the area.
[Figure 3E] Comparison map of spike amplitude (µV) in organoids with and without PFD addition. Scale bar is 0.5 mm.
[Figure 3F] The functional contact area (mm²) based on spike amplitude maps increases with PFD addition.
[Figure 3G] The bars in each graph, from left to right, show a comparison of the results before and after PFD addition, immediately after washing, and the day after washing. The graphs show, from left to right, (i) interburst interval, (ii) mean firing rate, and (iii) burst duration. No significant differences were observed in interburst intervals and burst durations, whereas significant differences were observed in mean firing rates.

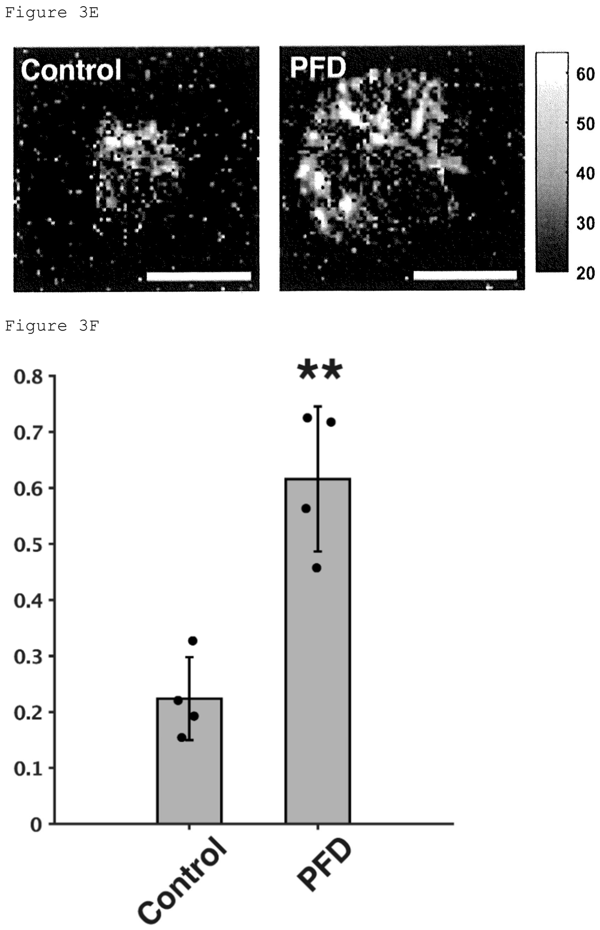

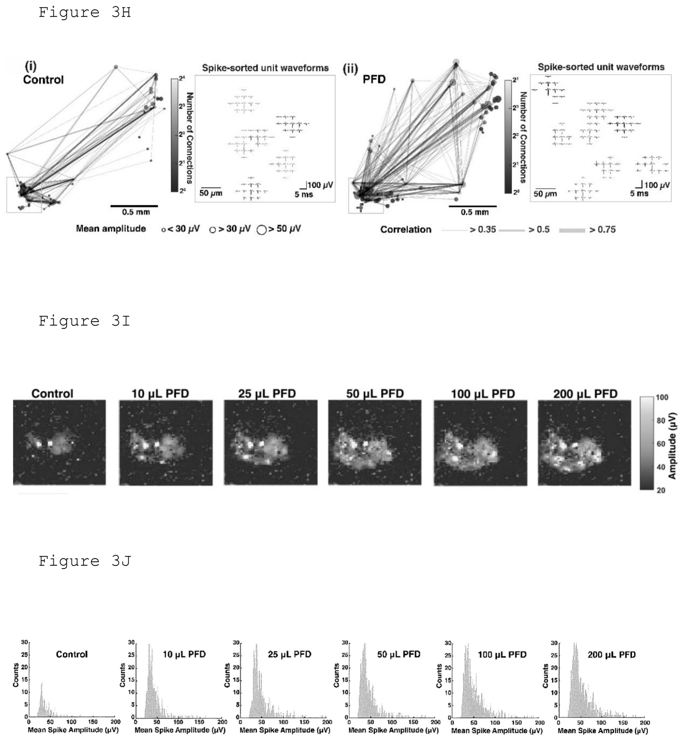

[Figure 3H] Functional connectivity map based on pairwise spike correlations between spike-sorted units in organoids. (i) is a comparative example without PFD addition. (ii) is with PFD addition. The dots in the figures represent the spatial location of a single unit on the HD-MEA. The size of the dots indicates the mean spike amplitude, and the density represents the number of functional connectivities found. Gray lines represent functional connections between single units, and the thickness of the lines indicates the strength of correlation. The right panels of (i) and (ii) plot the extracellular spike waveform trajectories of spike-sorted units selected from the boxed area at the bottom left of the respective left panels.
[Figure 3I] Changes in spike amplitude maps due to the influence of PFD amount. Spike amplitude maps of organoids without PFD and with different amounts of PFD. Scale bar is 500 µm.
[Figure 3J] Changes in histograms of mean spike amplitudes of active electrodes due to the influence of PFD amount. Histogram of the mean spike amplitude of organoids without PFD and with different amounts of PFD.
[Figure 3K] Records when PFD is added to organoids immediately after they are placed on HD-MEA. (A) Active electrode map with and without PFD. Active electrodes are white, inactive electrodes are black. Scale bar 0.5 mm. (B) The number of detected active electrodes is significantly greater when PFD is added than under the control condition without PFD. (C) Spike raster plot. This plot shows the time when spike activity is detected at each electrode (channels) over time. When PFD is added, more electrodes detects neural activity than under the control condition without PFD.
[Figure 4A] Activity map (center) and its enlarged view (right) from axon bundle recording analysis of motor neuron organoids with PFD addition.
[Figure 4B] Bandpass filtered signal of axon bundle recording on channel 11 in the right panel of Figure 4A. The vertical axis is amplitude (µV).
[Figure 4C] Example of an action potential waveform on channel 11 in the right panel of Figure 4A. The center line is the mean. The vertical axis is the amplitude (µV).
[Figure 4D] Active potentials propagating along the axon bundle, caused by the neural activity of selected channels. Channels 1 to 11 are shown from top to bottom. Each division on the horizontal axis is 50 ms, and each division on the vertical axis is 50 µV.
[Figure 4E] Histogram of spike counts versus stimulation timing (gray area) during optogenetic stimulation. The vertical axis represents spike counts.
[Figure 5A] (i) Map of active electrodes (scale bar is 0.5 mm) and (ii) distribution of mean spike amplitude (µV) when different perfluorocarbon compounds having 5 or more carbon atoms are applied to cortical organoids. Tested different perfluorocarbon compounds having 5 or more carbon atoms are perfluorohexane (PFH, C₆F₁₄), perfluoroheptane (PFHept, C₇F₁₆), perfluorooctane (PFO, C₈F₁₈), and perfluorododecalin (PFD, C₁₀F₁₈).
[Figure 5B] Comparison of the number of active electrodes between different perfluorocarbon compounds having 5 or more carbon atoms.
[Figure 5C] Comparison of mean spike amplitudes at active electrodes between different perfluorocarbon compounds having 5 or more carbon atoms.

### Description of Embodiments

Hereinafter, the present invention is described in detail.

The present invention is a method for measuring electric signals of cell tissue, the method comprising adding a perfluorocarbon compound having 5 or more carbon atoms and measuring electric signals of the cell tissue.

The cell tissue to be measured can be any cell tissue, regardless of whether it emits an electric signal or not. There are various methods for measuring electric signals, as described in the Background Art section, and in all of these methods, the addition of the perfluorocarbon compound having 5 or more carbon atoms improves the measurement sensitivity of the electric signal. Preferably, the measurement of electric signals of cell tissue is performed using an electrode array.

Also, it is more effective and preferable that the adding the perfluorocarbon compound having 5 or more carbon atoms be adding by replacing a culture solution.

Furthermore, the present invention is an electric signal enhancer for cell tissues, comprising a perfluorocarbon compound having 5 or more carbon atoms.

The hydrocarbon portion of the perfluorocarbon compound can be straight, branched, or alicyclic, and can be saturated or unsaturated. There are no particular limitations as long as the number of carbon atoms is 5 or more. Examples of straight or branched perfluorocarbon compounds having 5 or more carbon atoms which are easily available include perfluorohexane, perfluoroheptane, and perfluorooctane, and examples of alicyclic perfluorocarbon compounds having 5 or more carbon atoms which are easily available include perfluorodecalin.

### Examples

Hereinafter, the present invention is specifically described by way of Examples below, but the present invention is not limited to these Examples.

In Examples, three types of cell tissues were fabricated: hippocampal neurons, cortical organoids, and motor neuron organoids, and the electric signals of each cell tissue were measured. In addition, electric signals of cell tissue were measured using, as enhancers, perfluorocarbon compounds having 5 or more carbon atoms; namely perfluorohexane, perfluoroheptane, perfluorooctane, and perfluorodecalin. It is expected that much knowledge can be gained from analyzing the results of measuring electric signals of the fabricated tissue cells. However, the improvement in the sensitivity in measuring electric signals is not limited only to those on these cell tissue, but can be applied to any cell.

### <PDMS Culture Chip Fabrication>

A polydimethylsiloxane (hereinafter referred to as "PDMS") culture chip was fabricated by well-known procedures. First, a master mold was fabricated, and a PDMS device was fabricated using a mask. The master mold was fabricated by pouring negative photoresist SU-8 2100 or 2075 (manufactured by Nippon Kayaku Co., Ltd.) onto a silicon wafer and spin coating at 1200-1500 rpm for 30 seconds. The wafer was then baked for 9 minutes at 65°C and for 40 minutes at 95°C on a hotplate.

Then, UV light (365 nm, 2.5-3.0 mW/cm²) was irradiated using a photomask with desired pattern for 60-75 seconds. The wafer was baked for 7 minutes at 65°C and for 13 minutes at 95°C on a hotplate. After cooling, the wafer was developed with SU-8 developer for 15 minutes and washed with isopropyl alcohol (IPA) for 3 times. The wafer was baked for 3 minutes at 150°C in an oven. The culture chip was fabricated using a PDMS silicone elastomer kit, Sylgard 184 (manufactured by Dow Corning). Silicone elastomer and a curing agent were mixed at a weight ratio of 10:1, and the mixture was degassed, poured onto the master mold, and cured in the oven at 80°C overnight. PDMS construct was cut out using a scalpel, and holes for the organoids were punched with a 1.5 mm biopsy punch.

### <Cell Plating>

For primary hippocampal neurons, HD-MEA chips were precoated with 0.07% polyethyleneimine (PEI) in 50 mM borate buffer (pH 8.2) and cultured at 37°C and 5% CO₂ for 1 hour. The chips were washed 3 times with sterile water and incubated in 0.02 mg/ml laminin (feeding medium (FM)) at 37°C and 5% CO₂ for 1 hour. 100k dissociated cells were plated onto the chip area at a cell density of 2000 cells/mm². The cells were cultured in FM at 37°C and 5% CO₂, and the medium was replaced every 3-4 days during the culture period.

For motor neuron organoids (MNOs) and cortical organoids, the sensing area of the HD-MEA chip was coated with Matrigel in a medium DMEM/F12 (1:50) supplemented with HEPES (hydroxyethylpiperazineethanesulfonic acid) buffer and cultured at room temperature for 1 hour. The organoids were placed in the center of the electrode surface and cultured in NMM (neural maintenance medium) at 37°C and 5% CO₂. Cortical organoids were plated onto MEAs after 3-4 months of maturation, and motor neuron organoids were plated after 2 weeks. The medium was replaced every 3-4 days during the culture period.

For recording, HD-MEAs were also coated with Matrigel in DMEM/F12 (1:50) supplemented with HEPES and cultured at room temperature for 1 hour. After replacing the coating solution with RM (recording medium), the cortical organoids were placed on the MEAs. For motor neuron organoid recording, the PDMS culture chip in the well was detached from the bottom using forceps and held. To detach the MNOs from the PDMS constructs, a stream of culture solution was added to the wells by pipetting. Once the MNOs were suspended, they were picked up using a wide-bore pipette tip coated with 2% serum albumin (BSA) in phosphate-buffered saline (PBS) and transferred onto the MEA surface.

### <Electrophysiological Recordings>

For the experiment, a CMOS-based HD-MEA (MaxOne (manufactured by Maxwell Biosystems)) containing 26,400 platinum electrodes covering a sensing area of 3.85 × 2.10 mm² was used. The microelectrodes have a diameter of 7.5 µm and a center-to-center distance of 17.5 µm. The system includes 1024 configurable low-noise readout channels and records at a sampling rate of 20 kHz.

All recording were performed inside a cell culture incubator at 37°C and 5% CO₂ for 24 hours after media replacement using the MaxLab Live Software (v.20.1.6. (manufactured by MaxWell Biosystems)). First, an activity scan assay was performed, in which multiple defined areas of the MEA were recorded 365 times for 30 seconds. In 2-dimensional primary cell cultures, 7 electrode configurations with a total of 6600 electrodes at a pitch of 35 µm covering the entire sensing area were scanned. For organoid recording, images of the organoid locations on the chip were acquired and electrodes were scanned by manually selecting organoid-covered electrodes at 17.5 µm pitch. Scanning result were used to identify active electrodes based on spike frequency and spike amplitude and readout channels were routed to the 1024 most active electrodes.

### <PFD addition>

The medium was slowly removed until the meniscus of the medium touched the culture surface or the bottom of the cells. The removed conditioned medium was stored so that it could be added again to the cells after PFD administration. 100 µl of warmed PFD was added slowly and gently down from the side of the well using a 200 µl pipette tip. After the experiment was completed, the PFD was slowly removed from the end of the tip while the pipette tip was kept in vertical contact with the bottom of the tip surface. After removing PFD, the stored conditioned medium was added back to the cells.

### <Optogenetic Stimulations>

To stimulate organoids, optogenetic technology was used. At least 1 week before the experiment, 1 µl of AAVCAG-hCHR2-tdTomato (manufactured by VectorBuilder Inc.) was added to 1 ml of medium. For the stimulation, fiber-coupled LED of 470 nm (M470F3 - 470 nm, 17.2 mW (Min) Fiber-Coupled LED, 1000 mA, (manufactured by Thorlabs)) were controlled by T-cube LED driver (LEDD1B, 1.2A, (manufactured by Thorlabs)). A multimode fiber (0.22 NA, High-OH, 105 µm diameter core, 250-1200 nm (manufactured by Thorlabs)) was connected to the LED and pierced through the coin-shaped PDMS lid. The lid was placed on the electrode chip and aligned so that the tip of the fiber was positioned over the organoid. TTL pulses of 200 ms at 0.1 Hz were generated and sent by an Arduino Uno Rev3 device to control the LED driver.

### <Data Analysis>

The collected data was analyzed using MATLAB (R) 2018b (manufactured by MathWorks). The recorded signals were band-pass filtered within the frequency range of 300-3000 Hz, and 9 peaks-both positive and negative-with absolute values exceeding five times the standard deviation were identified as spikes.

### <Immunohistochemistry and Microscopy>

Cells and tissues were fixed with 4% paraformaldehyde for 1 hour and washed 3 times with PBS. Tissues were permeabilized for 30 minutes in PBS containing 0.1% Triton-X and washed 3 times with PBS. After that, cells were blocked in PBS containing 2% goat serum at room temperature for 1 hour. Tissues were then incubated overnight at 4°C in primary antibody in PBS containing 2% goat serum. After rinsing 3 times with PBS, cells were incubated in secondary antibody in PBS containing 2% goat serum at room temperature for 2 hours. Cell nuclei were labeled by incubating cells in PBS containing 1:1000 Hoechst solution for 10 minutes, followed by 3 times of rinses with PBS.

### <Statistics>

All analyses were performed in at least 3 replicates. All statistical tests and graphical presentations were performed using MATLAB (R) 2018b. Comparisons between 2 sample groups were performed using the Student's t-test using the function ttest(). Comparisons between 2 or more sample groups were performed using one-way analysis of variance (ANOVA) with the function anova1(), followed by a Tukey-Kramer post-hoc test for pairwise comparisons with the function multcompare().

### Example 1

### <Hippocampal Neurons>

Hippocampal neurons were obtained from ICR mouse embryos at embryonic day 17 (CLEA, Japan). Specifically, hippocampal cells were extracted from mice, washed 3 times with HBSS 085-09355 ((Hanks' Balanced Salt Solution) manufactured by Wako), and then incubated with trypsin and DNase for 10 minutes at room temperature. The cells were then washed 3 times with HBSS and dissociated in FM consisting of Neurobasal medium (manufactured by Thermo Fisher Scientific, 21103049) supplemented with 2% (v/v) B27 supplemented with vitamin A, 0.25% (v/v) GlutaMAX (manufactured by Thermo Fisher Scientific, 35050061), and 1% (v/v) PSA (manufactured by Sigma, A5955). The single cells were then centrifuged at 250 g for 5 minutes and resuspended in FM. 300k cells per well were seeded onto culture-treated 24-well plates and treated with PLL in 50 mM borate buffer (pH 8.2) at 37°C and 5% CO₂ for 1 hour. Cells were cultured in FM. The cells were maintained at 37°C and 5% CO₂, and the medium was replaced every 3-4 days during the culture period.

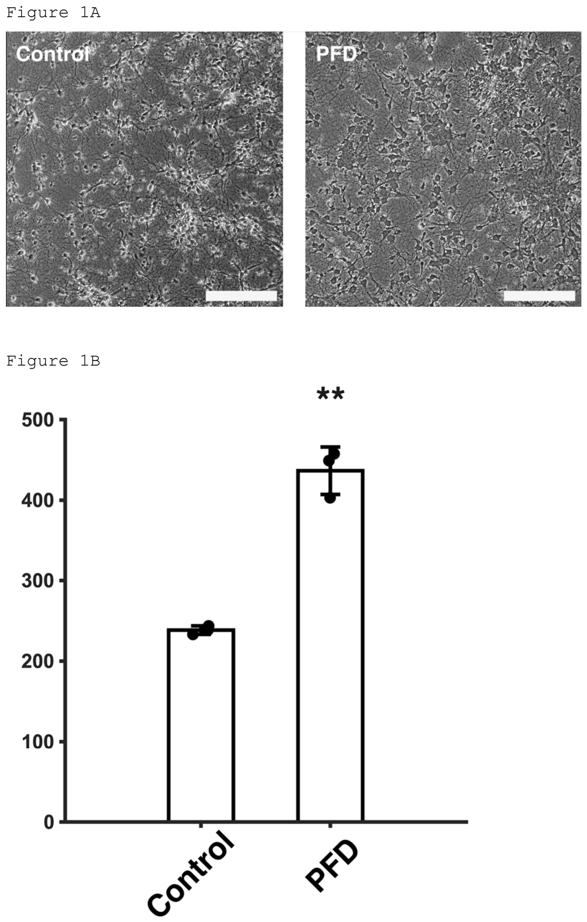

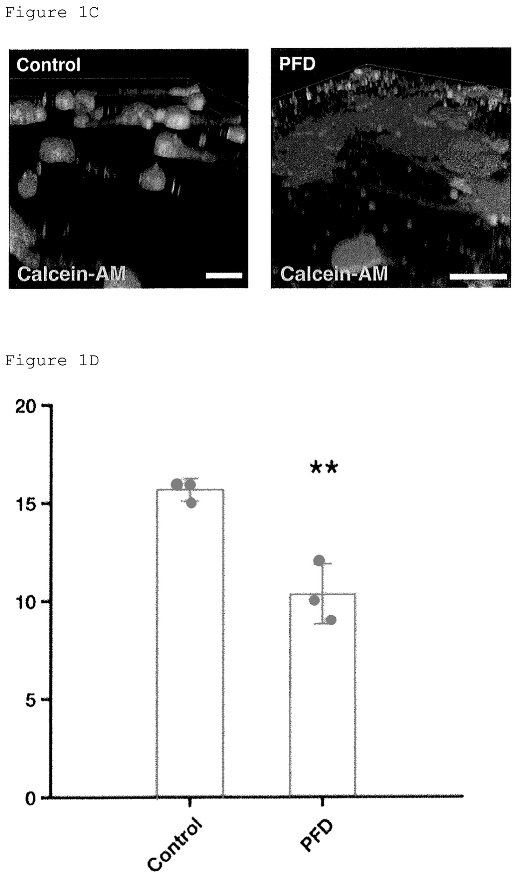

Primary hippocampal neurons obtained from mice were cultured in vitro. When the culture solution was removed and PFD was cast onto the neurons, the surface area of the cell bodies observed under a microscope significantly increased (see Figures 1A and 1B (Control is a comparative example before PFD addition; the same applies below)). Furthermore, labeling the cells with calcein-AM and observing them by confocal imaging further supported these observations. 3-dimensional reconstructions of neurons imaged under PFD condition showed a decrease in cell body height (see Figures 1C, 1D), providing further evidence of the flattening effect induced by PFD.

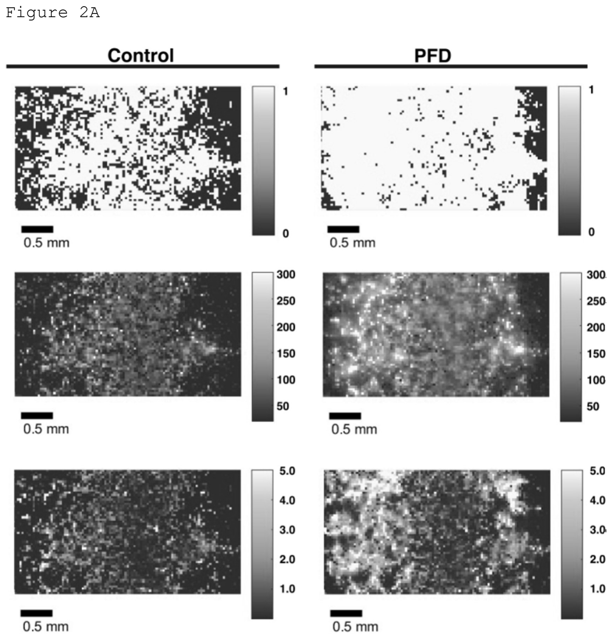

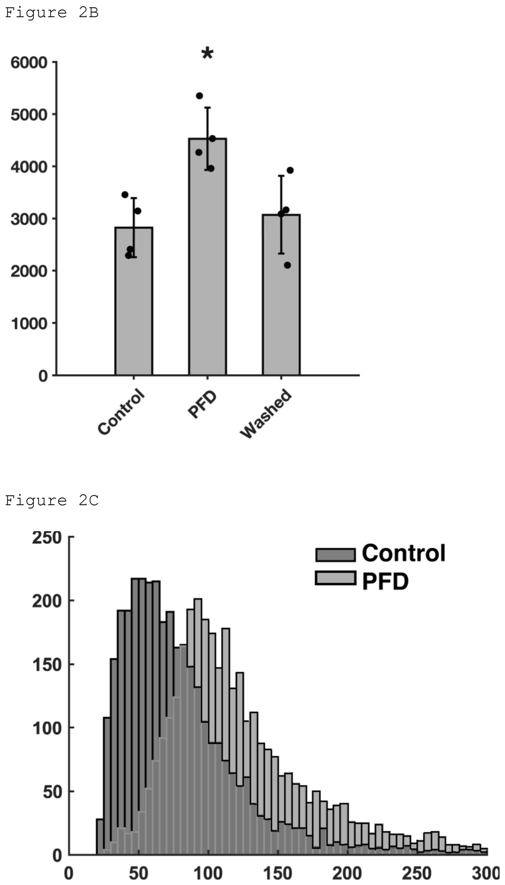

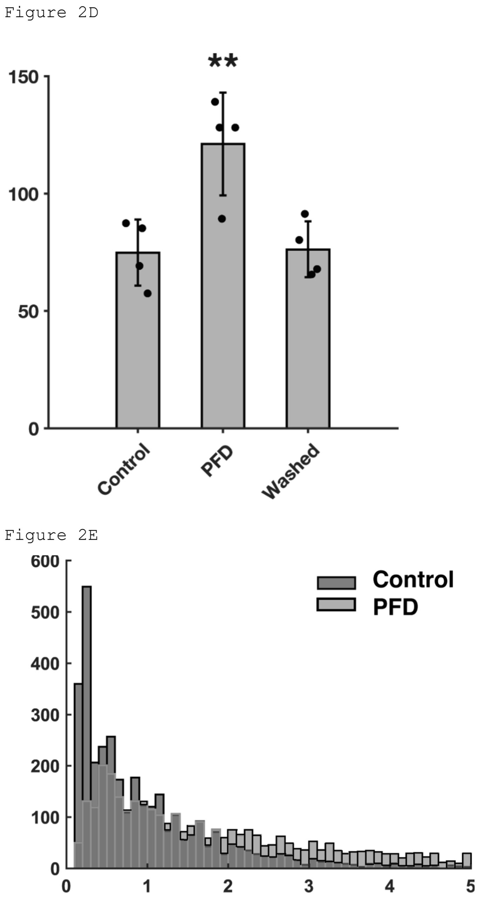

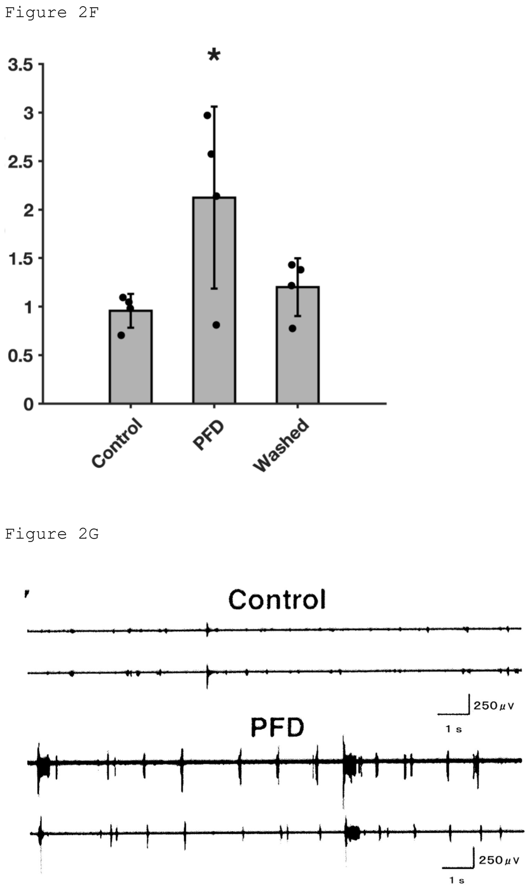

Primary hippocampal neurons were cultured on HD-MEA for 3 weeks and, in particular, neurons such as NeuN and βIII tubulin were stained with markers. Cells were recorded under both normal and PFD-added conditions. After recording, PFD was washed and the cells were reintroduced into the conditioned medium of the control group. Electrical readout of recordings with PFD showed enhanced activity compared to the pre-PFD state (see Figures 2A-2G). Specifically, when the entire MEA surface was scanned, significantly more active electrodes were observed under PFD condition than in the control group (see Figure 2A, top, and Figure 2B). Furthermore, when PFD was removed and the conditioned medium used before PFD recording was reintroduced, a similar number of active electrodes was detected as in the pre-PFD condition (see Figure 2B). MEA maps representing the mean spike amplitude across electrodes showed more and higher amplitudes in the PFD condition compared to the control condition (Figure 2A, middle, Figure 2D). The distribution of mean spike amplitudes across electrodes was skewed toward higher values in PFD condition, with the mean values being significantly higher than in the control (see Figure 2C). The mean spike rates were also significantly higher under PFD conditions (Figures 2E, 2F). Taken together, these results suggest that PFD addition improves recording performance, and its reversible effect is also achieved, as all parameters returned to baseline levels after washing.

### Example 2

### <Cortical Organoid>

Human iPS cell (hiPSC) lines were obtained from Kyoto University through the RIKEN BioResource Center cell bank (409B2, HPS0076). hiPS cells were cultured in mTeSR plus medium on tissue culture 12-well plates coated with human embryonic stem cell-compatible Matrigel. The medium was replaced every other day, and the cells were passaged when they were 80% confluent. Briefly, hiPSCs were washed with calcium- and magnesium-free PBS, lifted from the plate with ReLeSR reagent (manufactured by Stemcell Technologies), and seeded onto new Matrigel-coated wells.

Cortical organoids were cultured according to previously reported procedures. hiPS cells were dissociated from 70-80% confluent cells by incubating in TrypLE Express for 3 minutes under the condition of 37°C and 5% CO₂. The cells were resuspended in DMEM/F12 supplemented with HEPES and centrifuged at 250 g for 5 minutes. 10,000 cells per well were seeded onto mTeSR plus U-bottom ultra-low adhesion 96-well plates (Prime surface, manufactured by Sumitomo Bakelite) supplemented with 10 µM Y-23632. After 24 hours, the medium was replaced with neural induction medium (NIM) consisting of HEPES-containing DMEM-F12, 15% (v/v) knockout serum replacement, 1% (v/v) minimal essential medium non-essential amino acids (MEM-NEAA), and 1% (v/v) Glutamax, supplemented with 100 nM LDN-193189, 10 µM SB431542, and 5% (v/v) heat-inactivated FBS. On day 2, NIM was replaced with new NIM without adding FBS, and was replaced every other day until day 10. From days 10 to 18, the medium was replaced every other day with neural differentiation medium 1 (NDM1), which consisted of a 1:1 mixture of DMEM/F12 with HEPES and Neurobasal medium, 0.5% (v/v) N2 supplement, 1% (v/v) B27 supplement without vitamin A, 1% (v/v) Glutamax, 0.5% (v/v) MEM-NEAA, 0.25 mg/ml human insulin solution, and 1% (v/v) penicillin/streptomycin/aphotericin (PSA) (Sigma, A5955). On day 18, the medium was replaced with neural differentiation medium 2 (NDM2). The medium consisted of Neurobasal medium, 0.5% (v/v) N2 supplement, 1% (v/v) B27 supplement with vitamin A, 1% (v/v) Glutamax, 0.5% (v/v) MEM-NEAA, 0.25 mg/ml human insulin solution, 200 mM ascorbic acid, and 1% (v/v) PSA, supplemented with 20 ng/ml brain-derived neurotrophic factor (BDNF). From day 28 onwards, at each medium replacement, the medium was gradually (25% steps) switched to recording medium (RM) consisting of Brainphys medium supplemented with 2% (v/v) B27 supplement with vitamin A, 1% (v/v) Glutamax, 1% (v/v) PSA, and 20 ng/ml BDNF. Cells were maintained at 37°C and 5% CO₂, and NMM medium replacements were performed every 3-4 days during the organoid culture period.

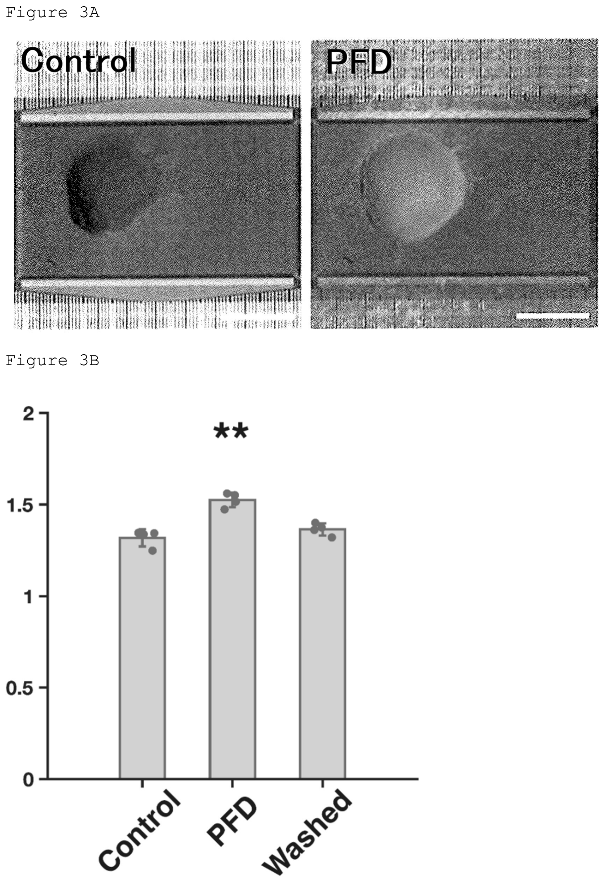

The effects of PFD in 3-dimensional neural tissue models, particularly cortical organoids, were evaluated. These organoids were fabricated using hiPSCs, cultured for 4 months, plated on HD-MEA, and then experimented in both the absence and presence of PFD. The flattened morphology observed in 2-dimensional culture was also evident in the 3-dimensional tissue. A comparison of organoid diameters observed from the top of the chip before and after PFD introduction revealed a significant increase in size which returned to the size before introducing PFD upon removal of PFD (see Figures 3A and 3B).

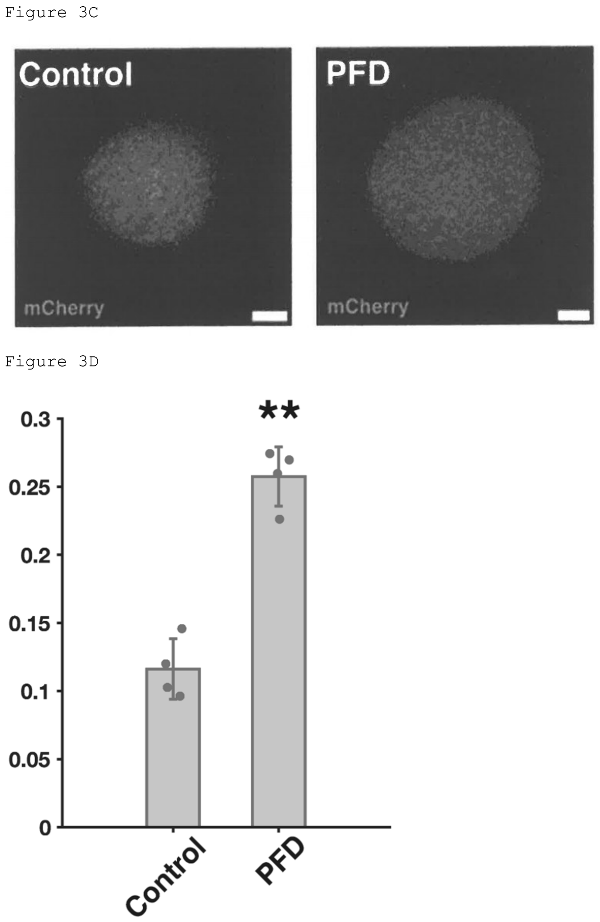

To investigate changes in the contact area with the MEA, organoids were fabricated from an iPS cell line expressing mCherry and plated in a well plate. Confocal microscopy images of fluorescent organoids taken from below the culture plate showed an increased surface diameter at the focal plane where the cells met the bottom of the culture dish, indicating an increased surface contact area in the presence of a PFD (see Figure 3C). The organoids' electrical footprints obtained in electrophysiological recordings confirmed that PFD application increased the contact area between organoids and MEA (see Figure 3D).

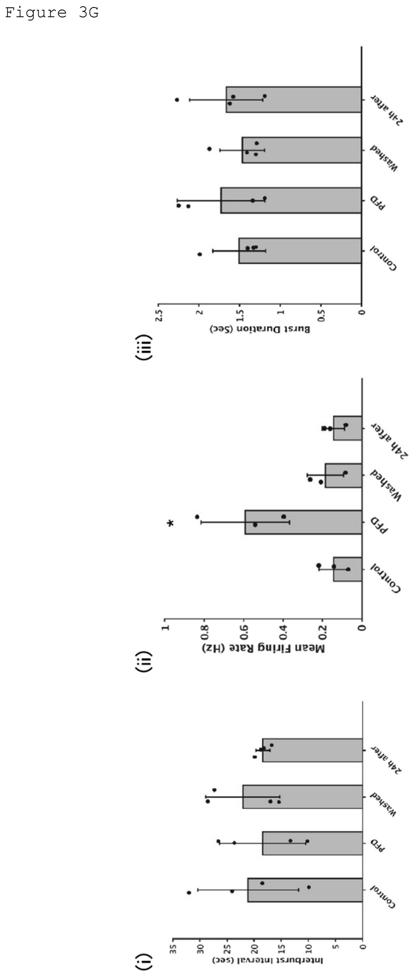

Both under normal condition and in the presence of PFD, the organoids showed consistent network-wide bursting behavior, with periods of silence between each burst. Recordability and network parameters were evaluated before the experiment, during PFD application, after removal of PFD, and 1 day after the experiment (see Figure 3G). Recordability parameters, such as the change in the number of active electrodes, the change in the number of spikes during a burst, and the mean spike amplitude, were significantly elevated under the PFD condition compared with pre-PFD recordings (see Figure 3B). These values returned to baseline levels after removal of PFD and 1 day after the experiment, indicating the reversibility of the enhancement effect on recording performance.

Furthermore, PFD addition resulted in the emergence of new spike-sorted units and increased correlations between spike pairs of single units in the analysis of functional connectivity between spike-sorted units calculated based on pairwise spike correlations using the Spike Time Tiling Coefficient (STTC; see Cutts, C. S. & Eglen, Detecting Pairwise Correlations in Spike Trains: An Objective Comparison of Methods and Application to the Study of Retinal Waves, Journal of Neuroscience 34, 14288-14303 (2014)), revealing many connections between cells within the organoid (see Figure 3H).

Furthermore, under the same conditions as above, the amount of PFD added was changed to 10, 25, 50, 100, and 200 µl, of which the effects were also examined.

The HD-MEA used was the same MaxOne manufactured by MaxWell Biosystems as above (https://www.mxwbio.com/wp-content/uploads/2024/10/MaxOne-Brochure-online-version.pdf) which is circular, having a diameter of 19 mm and a height of 8 mm.

Basically, the amount of PFD should be sufficient to cover the organoids. Adding at least 10 µl, preferably 50 µl or more, and more preferably 100 µl or more, makes it possible to more successfully measure cell activity (see Figures 3I and 3J).

In addition, under the same conditions as above, measurements were also performed after adding PFD immediately after placing the organoids on HD-MEA.

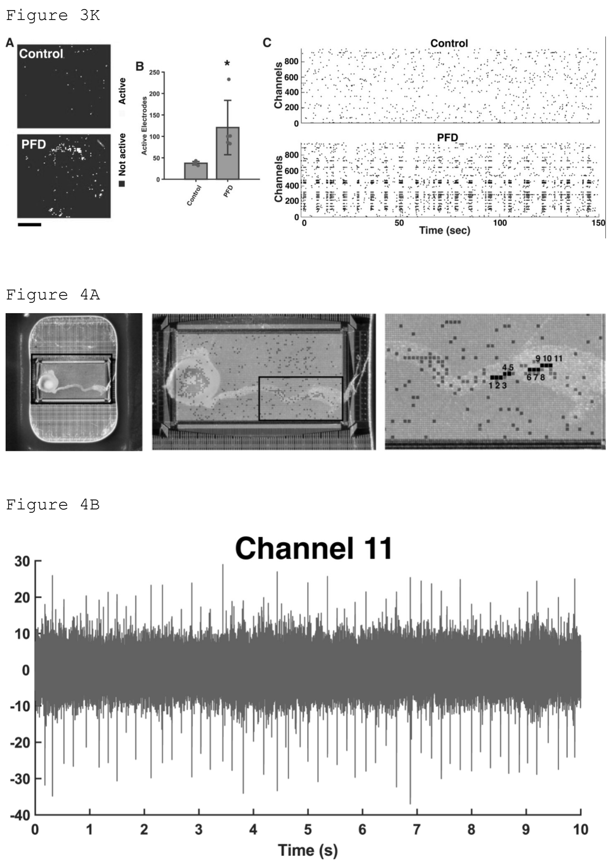

Even when PFD was added immediately after placing the organoids on MEA, it was possible that cell activity was more successfully measured than when no PFD was added (see Figure 3K).

### Example 3

### <Motor Neuron Organoids>

Motor spheroids were formed by known procedures. hiPSCs were dissociated from 70-80% confluent cells by incubating in TrypLE Express for 3 minutes under the condition of 37°C and 5% CO₂. The cells were resuspended in DMEM/F12 supplemented with HEPES and centrifuged at 250 g for 5 minutes. 40,000 cells per well were seeded onto mTeSR plus 7 U-bottom ultra-low binding 96-well plates (Prime surface, manufactured by Sumitomo Bakelite) supplemented with 10 µM Y-23632. After 24 hours, the medium was replaced with NIM supplemented with 100 nM LDN-193189 and 10 µM SB431542 for 2 days.

The base medium was gradually switched to N2 medium (N2M) consisting of Neurobasal medium, 1% (v/v) N2 supplement, 1% (v/v) GlutaMAX, and 1% (v/v) PSA. Specifically, the base medium ratio NIM:N2M was changed every other day on day 2 (4:0), day 4 (3:1), day 6 (1:1), day 8 (1:3), and day 10 (0:4). From day 2 to day 6, the base medium was supplemented with 10 µM SB431542, 100 nM LDN-193189, 5 µM DAPT, 5 µM SU5402, 1 µM RA, and 1 µM SAG. From day 6 to day 12, the medium was supplemented with 5 µM DAPT, 5 µM SU5402, 1 µM RA, and 1 µM SAG. After 12 days of culture, the medium was replaced with neural maintenance medium (NMM) consisting of Neurobasal medium supplemented with 2% (v/v) B27 supplement with vitamin A, 1% (v/v) Glutamax, 1% (v/v) PSA, and 20 ng/ml BDNF. The cells were maintained at 37°C and 5% CO₂, and the NMM medium was replaced every 3-4 days during the culture of 305 spheroids.

Custom-made PDMS culture chips were disinfected by immersion in 70% ethanol, allowed to dry, and then attached to the bottom of wells of a culture-treated 12-well plate. The inner channels of the chip were coated with Matrigel in DMEM/F12 plus HEPES solution (1:50) at room temperature for 1 hour. The coating solution was replaced with NMM, and a d12 motor spheroid was placed into one of the wells of the culture device using a wide-bore pipette tip. Axons from the spheroids began to extend to the surface. Due to the physical constraints of the PDMS culture chip, axons can only extend along the channels, and after 2 weeks of culture, they naturally form axon bundles. Successful formation of the axon bundle result in a motor neuron organoid.

As described above, to investigate the capability of this method in capturing axonal signals from bundled axons, motor neuron organoids were fabricated and electric signals upon PFD addition were measured.

The motor neuron organoids fabricated as described above were removed from the chip and placed on MEAs for acute recordings. The MEAs were scanned to determine active electrodes, and most of the detected active electrodes overlapped with organoid structures, including axon bundles, suggesting that the detected activity originated from neural tissue. Spike activity could be detected at multiple electrodes beneath the axon bundle, and in particular, the propagation of axonal activity could be visually tracked along the axon bundle.

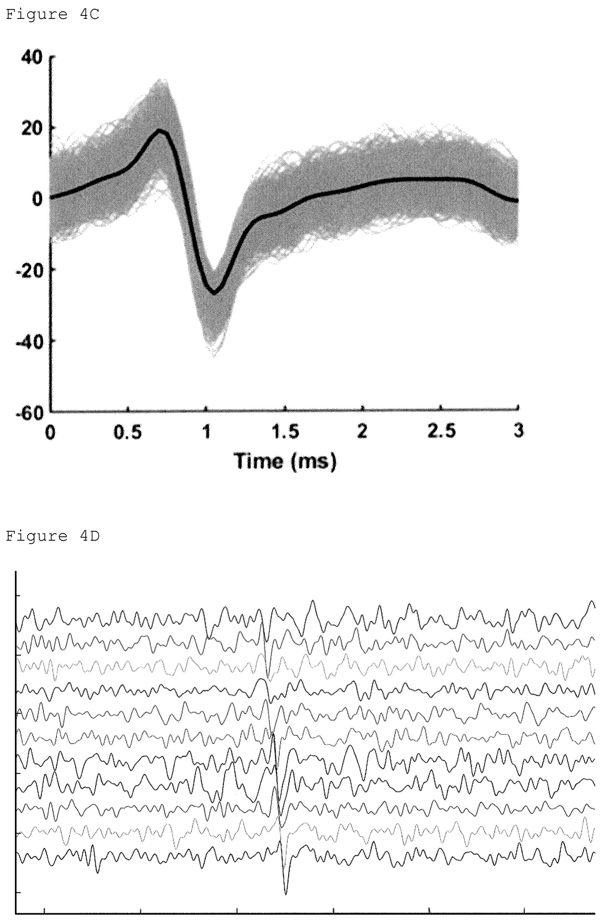

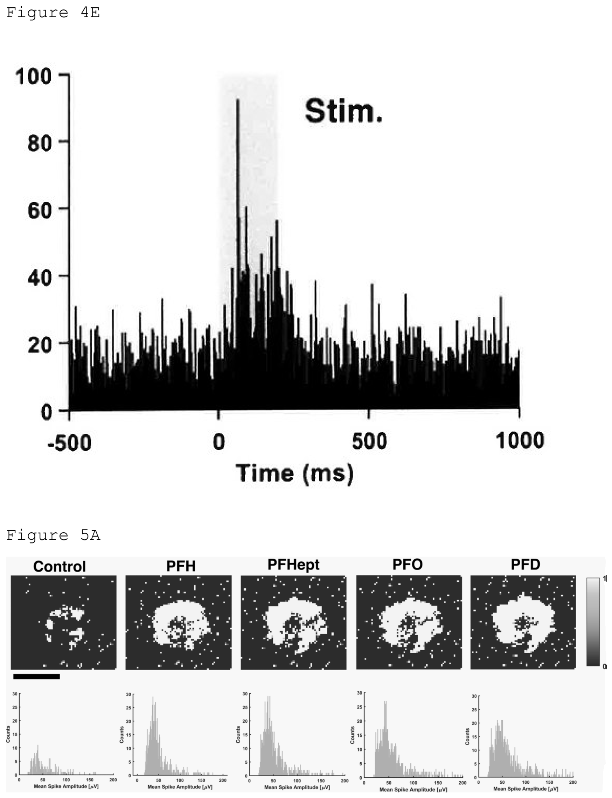

Motor neuron organoids were virally transfected to express channelrhodopsin (ChR2[H134R]) and subjected to optogenetic stimulation of the tissue. The organoid bodies of plated motor neuron organoids were stimulated every 10 seconds with 200 ms pulses of blue light (470 nm) using an optical fiber. When spike counts were superimposed at the timing of stimulation, it was found that spike activity increased during the time period when light was being irradiated. Furthermore, by investigating specific recording channels, it was easily observed that some stimuli evoked spikes in the organoids, followed by propagation of activity along the axon bundles (see Figures 4A-4E).

### Example 4

### <Perfluorocarbon compounds having 5 or more carbon atoms>

Then, using the cortical organoids described in Example 2 above, electric signals of cell tissue were measured using, as enhancers, perfluorohexane (PFH, C₆F₁₄), perfluoroheptane (PFHept, C₇F₁₆), perfluorooctane (PFO, C₈F₁₈), and perfluorododecalin (PFD, C₁₀F₁₈), excluding perfluorodecalin.

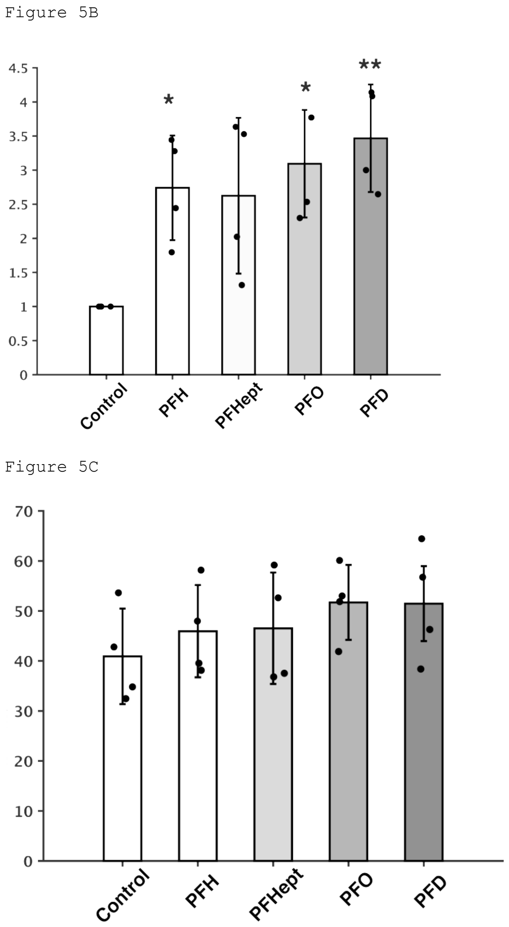

Clearly and significantly larger electric signals were obtained by all the compounds; perfluorohexane (PFH), perfluoroheptane (PFHept), perfluorooctane (PFO), and perfluorododecalin (PFD), than in the comparative example, in which culture solution was added. In particular, larger electric signals were obtained by perfluoroheptane (PFHept) and perfluorooctane (PFO) than by perfluorohexane (PFH), and by perfluorododecalin (PFD) than by perfluoroheptane (PFHept) and perfluorooctane (PFO) (see Figures 5A-5C). More specifically, significant results were obtained for the number of active electrodes, the distribution of the number of active electrodes, and the mean spike amplitude of the active electrodes.

These results indicated that perfluorocarbon compounds having 5 or more carbon atoms were effective in measuring electric signals in cell tissues and were also useful as an electric signal enhancer in cell tissues.

As described above, the increase in the number of active electrodes, spike amplitude, and mean spike rate under the condition of perfluorocarbon compounds having 5 or more carbon atoms indicates improved signal detection and reliability, which made it possible to monitor axonal signals from axon bundles, which is usually difficult to record. Furthermore, these effects are reversible upon removal of the PFD, allowing for safe application without adversely affecting subsequent recordings. Furthermore, this method is not only for permanently plated cells, but also makes it possible to record from temporarily stabilized tissues. Perfluorocarbon compounds having 5 or more carbon atoms can be simply cast, causing minimal damage to cells, and because they are colorless, tissues can be clearly observed. Additionally, the ability to observe the tissue is important when combining electrophysiology with optical techniques such as fluorescence imaging or optogenetic stimulation. In particular, optogenetic stimulation demonstrated in channelrhodopsin-expressing motor neuron organoids demonstrates the versatility of this approach and promises to be highly applicable to research of neural activity in complex 3-dimensional structures.

### Industrial Applicability

Measuring the electric signal cell tissue using perfluorocarbon compounds having 5 or more carbon atoms can be used for analyzing cell tissue.

## Claims

1. A method for measuring electric signals of cell tissue, comprising adding a perfluorocarbon compound having 5 or more carbon atoms.

2. The method for measuring electric signals of cell tissue according to claim 1, wherein the perfluorocarbon compound is perfluorodecalin.

3. The method for measuring electric signals of cell tissue according to claim 1 or 2, wherein the measurement of electric signals of cell tissue is performed using an electrode array.

4. The method for measuring electric signals of cell tissues according to claim 1 or 2, wherein the adding the perfluorocarbon compound is adding by replacing a culture solution.

5. An electric signal enhancer for cell tissues, comprising a perfluorocarbon compound having 5 or more carbon atoms.

6. The electric signal enhancer for cell tissue according to claim 5, wherein the perfluorocarbon compound is perfluorodecalin.
